# EUROPEAN PATENT APPLICATION

(11) **EP 3 756 551 A1**
(43) Date of publication of application: **30.12.2020**
(21) Application number: 19756944.5
(22) Date of filing: 21.02.2019
(51) Int. Cl.: A61B 8/00

(54) **BACKING MATERIAL, PRODUCTION METHOD THEREFOR, AND ACOUSTIC WAVE PROBE**

(30) Priority: 22.02.2018 JP 2018029899
(71) Applicant: Nisshinbo Holdings Inc., Tokyo 103-8650 (JP); Ueda Japan Radio Co., Ltd., Ueda-shi, Nagano 386-8608 (JP)
(72) Inventor: TOMITA, Hideshi, Chiba-shi, Chiba 267-0056 (JP); SUZUKI, Takashi, Chiba-shi, Chiba 267-0056 (JP); KOBAYASHI, Shigeo, Ueda-shi, Nagano 386-0032 (JP)
(74) Representative: Leifert & Steffan Patentanwälte PartG mbB
(86) International application number: PCT/JP2019/006494
(87) International publication number: WO 2019/163876

(57) **Abstract**

The present invention provides a backing material having an excellent attenuation effect of acoustic wave vibration, a method of producing the same, and an acoustic wave probe provided with the backing material. The backing material includes a resin and a magnetized particle, in which the magnetized particle has a magnetic flux density of 1,000 to 15,000 gauss.

## Description

### Technical Field

The present invention relates to a backing material, a method of producing the same, and an acoustic wave probe provided with the backing material of the present invention.

### Background Art

Generally, in ultrasonic diagnosis, ultrasonic waves are propagated into the inside of an object (living body) to receive an echo thereof, and a variety of diagnostic information including a tomographic image of the object is acquired on the basis of an echo receiving signal.

In such ultrasonic diagnosis, transmission/reception of ultrasonic waves is conducted through an acoustic wave probe. The acoustic wave probe is provided with a piezoelectric element (transducer) in charge of electroacoustic conversion. Furthermore, an acoustic matching layer and an acoustic lens are provided in this order on the ultrasonic transmission/reception surface side (object side) as seen from the piezoelectric element, whereas a backing material is provided on the back surface side (power supply side).

In such an acoustic wave probe, the backing material is provided for the purpose of not only holding the piezoelectric element but also acoustically braking it to suppress an excessive vibration, thereby shortening a pulse interval of the ultrasonic waves and improving a distance resolution in an ultrasonic diagnostic image. As characteristics required for such a backing material, there are (i) a sound wave is efficiently absorbed in the interior of the backing material; (ii) reflection on an interface between the piezoelectric element and the backing material is low; and so on.

In response to the aforementioned required characteristic (i), a technique for enhancing an attenuating effect of acoustic wave vibration in the interior of the backing material has hitherto been investigated. In addition, in response to the aforementioned required characteristic (ii), a technique for making an acoustic impedance of the backing material close to the piezoelectric element particularly in the vicinity of the interface, specifically, a method of increasing a packing ratio of a filler, a method of preventing sedimentation of a filler to make a homogenous composition, a method of using a high-density particle of ferrite, etc., and so on have been investigated.

For example, PTL 1 proposes a technology in which in order to provide a backing material having a homogenous composition by increasing a packing ratio of a filler and preventing sedimentation of the filler, a filler having a magnetic substance coated thereon is used and cured through impression of a magnetic field, thereby suppressing the sedimentation of the filler.

In addition, PTL 2 proposes a technology in which in order to provide a backing material having a high attenuation amount of acoustic wave vibration, having an appropriate acoustic impedance, and being hardly thermally deformed during dicing, a filler mixture and a nanocomposite epoxy resin are used.

However, the aforementioned techniques could not sufficiently respond to a requirement for more improvement of the attenuation amount of acoustic wave vibration in recent years.

### Citation List

### Patent Literature

PTL 1: JP 6-225392 A
PTL 2: JP 2011-176419 A

### Summary of Invention

### Technical Problem

Under the aforementioned circumstances, the present invention has been made, and an object thereof is to provide a backing material having an excellent attenuation effect of acoustic wave vibration, a method of producing the same, and an acoustic wave probe provided with the backing material of the present invention.

### Solution to Problem

The present inventors made extensive and intensive investigations. As a result, it has been found that when a backing material includes a resin and a magnetized particle, and the magnetized particle has a magnetic flux density of 1,000 to 15,000 gauss, a backing material which is excellent especially in an attenuation effect of acoustic wave vibration is provided, thereby leading to accomplishment of the present invention.

Specifically, the gist and constitution of the present invention are as follows.
[1] A backing material including a resin and a magnetized particle, wherein
   the magnetized particle has a magnetic flux density of 1,000 to 15,000 gauss.
[2] The backing material as set forth in the above [1], wherein the magnetized particle has an average particle diameter of 0.1 to 90 µm.
[3] The backing material as set forth in the above [1] or [2], wherein the magnetized particle is ferrite.
[4] An acoustic wave probe provided with the backing material as set forth any one of the above [1] to [3].
[5] A method of producing a backing material, including
   a step of obtaining a resin composition containing a liquid resin and a magnetic substance particle,
   a step of curing the resin composition to obtain a cured product, and
   a step of impressing a magnetic field on the cured product, to convert the magnetic substance particle into a magnetized particle, wherein
   the magnetized particle has a magnetic flux density of 1,000 to 15,000 gauss.
[6] The method of producing a backing material as set forth in the above [5], wherein the magnetic substance particle has a residual magnetic flux density of 1,000 to 15,000 gauss.

### Advantageous Effects of Invention

In accordance with the present invention, a backing material having an excellent attenuation effect of acoustic wave vibration, a method of producing the same, and an acoustic wave probe provided with the backing material of the present invention can be provided.

### Brief Description of Drawings

Fig. 1 is a diagrammatic perspective view showing a representative structure of an acoustic wave probe.
Fig. 2 is a view for explaining an attenuation effect evaluation method of a backing material.
Fig. 3 is a view for explaining a scattering evaluation method of an attenuation effect of a backing material.

### Description of Embodiments

Embodiments of a backing material and a method of producing the same according to the present invention are hereunder described in detail.

The backing material of the present invention is one including a resin and a magnetized particle, wherein the magnetized particle has a magnetic flux density of 1,000 to 15,000 gauss.

In view of the fact that the backing material of the present invention includes, as a filler, a magnetized particle having a predetermined magnetic flux density, a magnetic interaction is formed between the magnetized particles. When this interaction effectively enhances the attenuation effect of acoustic wave vibration due to the filler, the acoustic wave in the interior of the backing material can be efficiently absorbed.

The backing material of the present invention contains a resin and a magnetized particle having a predetermined magnetic flux density. In addition, the backing material of the present invention may contain, as other component, a component other than the resin and the magnetized particle within a range where the effects of the present invention are not impaired. The explanation is hereunder made in detail for every constituent component.

### (Resin)

In this specification, in the case of referring to simply as "resin", for example, the resin refers to one (resin cured product) obtained by curing an uncured liquid resin which will be explained in the production method of a backing material as mentioned later.

Although such a resin is not particularly limited, examples thereof include a silicone resin, a urethane resin, an epoxy resin, a nitrile butadiene rubber, and an isoprene rubber. Above all, a silicone resin and an epoxy resin are preferred from the standpoint of easiness of kneading in a state before curing.

Examples of the silicone resin include dimethyl silicone, methylphenyl silicone, phenyl silicone, and modified silicone. Above all, dimethyl silicone and methylphenyl silicone, each of which has flexibility after curing, are preferred. When such a silicone resin having flexibility after curing is used to form a backing material, it can also be used upon being bent in conformity with the shape of the probe. In addition, the silicone resin is preferably a cured product of an addition reaction type liquid silicone resin as mentioned later. Here, though the addition reaction type liquid silicone resin includes a one-pack type and a two-pack mixing type, in the case where the addition reaction type liquid silicone resin is of a one-pack type, and a curing agent is used at the same time, the cured product refers to the whole of a cured product resulting from curing of a mixture thereof. In addition, in the case where the addition reaction type liquid silicone resin is of a two-pack mixing type, the cured product refers to the whole of a material resulting from curing of a mixture of the two liquids.

The epoxy resin is preferably one having flexibility after curing, and above all, a rubber-modified epoxy resin and a long-chain epoxy resin are preferred. When such an epoxy resin having flexibility is used to form a backing material, it can also be used upon being bent in conformity with the shape of the probe. Here, the epoxy resin refers to the whole of a cured product resulting from curing of a mixture of an uncured liquid epoxy resin as mentioned later and a curing agent.

### (Magnetized Particle)

The magnetized particle plays a role as the filler. A high-density particle of ferrite, tungsten, or the like has hitherto been used for the filler. Such a high-density particle is dispersed in the resin, and an effect for attenuating an acoustic wave vibration propagating in the backing material is exhibited. As for a mechanism of the generation of attenuation of vibration due to the high-density particle, the following two are mainly considered. The attenuation is caused due to two actions that (1) because the particle has a high density, a large energy is required for vibration; and (2) because the high-density particle is vibrated more hardly than the surrounding resin, it is vibrated belatedly as compared with the resin, and an antiphase is generated in the vibration due to this belatedness, whereby the surrounding vibration is cancelled.

The present inventors made extensive and intensive investigations regarding the attenuation mechanism of vibration by the aforementioned high-density particle. As a result, from the viewpoint of making the high-density particle hard to vibrate, it has been found that it is effective to bring a magnetic interaction between the particles. That is, in view of the fact that the high-density particle to be dispersed in the resin is a magnetized particle having a predetermined magnetic force, the magnetic interaction works between the particles, whereby the aforementioned two attenuation actions can be efficiently enhanced.

On the basis of the aforementioned findings, it has been found that by using, as the filler to be contained in the backing material, a magnetized particle having a magnetic flux density of 1,000 to 15,000 gauss, a magnetic interaction can be sufficiently brought between the particles, and the attenuation effect of acoustic wave vibration can be more enhanced, thereby leading to accomplishment of the present invention.

In this specification, the "magnetized particle" is one resulting from magnetization of a magnetic substance particle and refers to a particle exhibiting a magnetic action (magnetic force).

The magnetic flux density of the magnetized particle is 1,000 to 15,000 gauss, preferably 1,100 to 10,000 gauss, and more preferably 1,200 to 5,000 gauss. When the magnetic flux density of the magnetized particle falls within the aforementioned range, a sufficient magnetic interaction can be brought between the particles, and the attenuation effect of acoustic wave vibration can be more enhanced. On the other hand, when the magnetic flux density of the magnetized particle is less than 1,000 gauss, the sufficient magnetic interaction cannot be brought between the particles. In addition, a magnetized particle having a magnetic flux density of more than 15,000 gauss is liable to cause a problem in handling properties of the material per se, and hence, such is not preferred.

Here, the magnetic flux density of the magnetized particle is considered to be substantially identical with a residual magnetic flux density of the magnetic substance particle to be used as a raw material as mentioned later. This value is adopted as a value (catalog value) of the residual magnetic flux density described in a product catalog of magnetic substance particle, and in the case where the value of residual magnetic flux density is not available from the foregoing catalog or the like, a value measured by a known method may also be adopted.

An average particle diameter of the magnetized particle is preferably 0.1 to 90 µm, more preferably 0.8 to 90 µm, and still more preferably 0.8 to 30 µm. By allowing the average particle diameter of the magnetized particle to fall within the aforementioned range, the kneading becomes easy, a good-quality backing material can be provided without containing an air bubble on the surface, and a good-quality attenuation effect of acoustic wave vibration is obtained. The average particle diameter of the magnetized particle is considered to be substantially identical with an average particle diameter of the magnetic substance particle to be used as a raw material as mentioned later.

Now, it has hitherto been general to use, as the filler, a particle having a relatively large diameter (hereinafter sometimes referred to simply as "large-diameter particle") in order to enhance the attenuation effect of acoustic wave vibration. This is because the large-diameter particle is large in an energy required for vibration and is able to make the attenuation of acoustic wave vibration large as compared with a particle having a relatively small diameter (hereinafter sometimes referred to simply as "small-diameter particle").

But, in recent years, in association with miniaturization of the acoustic wave probe, miniaturization of the piezoelectric element per se is also being advanced, and the corresponding backing material is also required to have homogeneity of the attenuation effect of acoustic wave vibration in a small range. For that reason, in the conventional backing materials, in the case of using a large-diameter particle as the filler particle, there is a tendency that unevenness in the density to be caused due to the large-diameter particle is liable to be generated, and scattering in the attenuation effect of acoustic wave vibration is liable to be generated between the elements. In contrast, from the viewpoint of reducing the scattering in the attenuation effect to be caused due to the unevenness in the density of the backing material, though a method for miniaturizing the filler particle may be considered, because as mentioned above, the small-diameter particle is inferior in the attenuation effect of acoustic wave vibration to the large-diameter particle, the sufficient attenuation effect of acoustic wave vibration as the backing material cannot be maintained.

In the light of the above, from the viewpoint of miniaturization of the element in recent years, it was difficult to provide a backing material which is small in the scattering in the attenuation effect while maintaining the attenuation effect well.

In contrast, according to the backing material of the present invention, by utilizing the magnetic interaction which the magnetized particle has, even when a magnetized particle having a relatively small diameter is used as the filler particle, an attenuation action of acoustic wave vibration can be efficiently enhanced, and an excellent attenuation effect of acoustic wave vibration is obtained. According to this, a backing material coping with miniaturization of the element, in which both maintenance of the attenuation effect of acoustic wave vibration and suppression of the scattering in the attenuation effect are made compatible with each other, can be provided.

From the viewpoint of reducing the scattering in the attenuation effect of the backing material, an average particle diameter of the magnetized particle is preferably 90 µm or less, more preferably 50 µm or less, and still more preferably 30 µm or less. By allowing the average particle diameter of the magnetized particle to fall within the aforementioned range, even when the element shape is miniaturized, the scattering in the attenuation effect of the backing material can be made small while maintaining the attenuation effect of acoustic wave vibration of the backing material well.

Examples of the magnetized particle include a particle of iron, cobalt, nickel, or an alloy thereof, ferrite, or the like. Above all, a ferrite particle which is able to give the aforementioned predetermined magnetic flux density, does not conduct, is chemically stable and high in density, and has a high coercive force is suitable. Examples of the ferrite particle include Ni-Zn-based ferrite and Mn-Zn-based ferrite.

The density of the magnetized particle is preferably 3.0 to 9.0 g/cm³, and more preferably 5.0 to 9.0 g/cm³. Such a magnetized particle is able to effectively attenuate the acoustic wave vibration as the high-density particle. The density is identical with a density of the magnetic substance particle as a raw material as mentioned later because it does not cause a volume change due to magnetization.

Although a shape of the magnetized particle is not particularly limited, examples thereof include a true sphere shape, an elliptical sphere shape, and a crushed shape.

The content of the magnetized particle is preferably 50 to 90% by mass, more preferably 67 to 89% by mass, and still more preferably 75 to 88% by mass in the backing material. By allowing the content of the magnetized particle to fall within the aforementioned range, the attenuation effect of acoustic wave vibration can be sufficiently exhibited. On the other hand, when the foregoing content is less than 50% by mass, the attenuation effect of acoustic wave vibration is not sufficiently obtained, whereas when it is more than 90% by mass, not only it requires time for kneading, but also the moldability tends to worsen.

### (Other Component)

The backing material may further contain other component than those as mentioned above, as the need arises. Examples of the other component include a coloring agent, a platinum catalyst, a curing accelerator, a curing retarder, a solvent, a dispersant, an antistatic agent, an antioxidant, a flame retarder, and a thermal conductivity enhancer.

The coloring agent is frequently blended for the purpose of discrimination or cleanliness confirmation, and examples of such a coloring agent include a pigment, such as carbon and titanium oxide, and a dye. These components may be used alone or may be used in combination of two or more thereof.

The curing accelerator is a component to be blended for the purpose of shortening a curing time, dropping a curing reaction temperature, or the like. Examples of such a curing accelerator include imidazoles. These components may be used alone or may be used in combination of two or more thereof.

### (Hardness)

In the backing material of the present invention, a hardness as measured with a type A durometer (hereinafter also referred to as "type A hardness") in conformity with JIS K6253-3:2012 is preferably 50 to 95, more preferably 60 to 95, and still more preferably 70 to 95. When the type A hardness falls within the aforementioned range, the shape retention characteristics as the backing material become favorable. In particular, taking into consideration deformation or fracture on the practical use as well as attenuation characteristics, the type A hardness is still more preferably 70 to 95.

### (Density)

A density of the backing material is preferably 1.7 to 5.0 g/cm³, more preferably 2.3 to 4.7 g/cm³, and still more preferably 2.8 to 4.5 g/cm³. When the density falls within the aforementioned range, an excellent acoustic impedance required for the backing material is revealed, and a favorable packing material is provided. In this specification, the density of the backing material means a value as measured by the method described in the section of Examples.

### (Attenuation Effect of Acoustic Wave Vibration)

The attenuation effect of acoustic wave vibration as the backing material can be, for example, evaluated in terms of an attenuation factor of the acoustic wave as mentioned later. In the backing material, the aforementioned attenuation factor is preferably 4.5 or more, and more preferably 6.0 or more. So far as such an attenuation factor is concerned, an excellent attenuation effect of acoustic wave vibration as the backing material is exhibited. A specific measurement method of attenuation factor is described on the pages of the section of Examples.

### [Production Method of Backing Material]

An example of a preferred method of producing a backing material of the present invention is hereunder described. It should be construed that the backing material of the present invention is not limited by the following production method.

The production method of a backing material of the present invention includes
a step of obtaining a resin composition containing a liquid resin and a magnetic substance particle,
a step of curing the resin composition to obtain a cured product, and
a step of impressing a magnetic field on the cured product, to convert the magnetic substance particle into a magnetized particle, wherein
the magnetized particle has a magnetic flux density of 1,000 to 15,000 gauss.
The production method is hereunder described in detail.

### (Step of Obtaining a Resin Composition)

First of all, the following liquid resin and magnetic substance particle, and optionally other component are prepared, respectively, and appropriate amounts thereof are weighed in predetermined blending ratios. The weighing can be performed by a known method, the blending ratios of the respective components follow the contents in the aforementioned backing material unless otherwise specifically indicated.

Here, the liquid resin refers to a resin material having appropriate fluidity and is one capable of being cured through a curing reaction or the like, to form a cured product having a hardness to a degree at which a fixed shape can be retained. Examples of such a liquid resin include a silicone resin, a urethane resin, an epoxy resin, a nitrile butadiene rubber, and an isoprene rubber. Above all, an addition reaction type liquid silicone resin and a liquid epoxy resin are preferred.

Here, the addition reaction type liquid silicone resin means a liquid silicone resin which is cured through an addition reaction. In general, the liquid silicone resin is classified into an addition reaction type and a condensation reaction type according to the kind of curing reaction. Here, the condensation reaction type is concerned with a case where a low-molecular compound (for example, acetone or an oxime) is produced as a desorbed component during the curing reaction and vaporized to form an air bubble in the backing material. Such an air bubble occasionally contributes to formation of a structure giving an influence against the acoustic absorption in the interior of the backing material, and thus, such is not preferred. For that reason, the liquid silicone resin is desirably one which does not produce the desorbed component in the curing reaction, and an addition reaction type liquid silicone resin is suitable. Such an addition reaction type liquid silicone resin is, for example, corresponding to one having hydrogen or a vinyl group.

The addition reaction type liquid silicone resin is not particularly limited, and known materials can be broadly used, and any of experimental synthetic products and commercially available products may also be used. In addition, the addition reaction type liquid silicone resin includes a one-pack type and a two-pack mixing type, and any of these types can be used.

As the aforementioned addition reaction type liquid silicon resin, there can be exemplified "KE-1031 A/B", "KE-109E A/B", and "KE-103", all of which are available from Shin-Etsu Chemical Co., Ltd.; and "EG-3000", "EG-3100", "EG-3810", "527", and "S1896FREG", all of which are available from Dow Corning Toray Co., Ltd.

Here, though the one-pack type addition reaction type liquid silicone can be cured even without using a curing agent, a curing agent may be added as the need arises. By adding the curing agent, the hardness can be increased, or curing is accelerated, whereby a curing time can be shortened.

The curing agent is not particularly limited so long as it is able to cure an uncured liquid silicone resin through an addition type reaction, and known materials can be broadly used, and any of experimental synthetic products and commercially available products may also be used. Examples thereof include "C-8B" available from Shin-Etsu Chemical Co., Ltd.; and "RD-7" available from Dow Corning Toray Co., Ltd.

Although a blending amount of the curing agent is not particularly limited, it is preferably 0.1 to 10 parts by mass, and more preferably 0.1 to 5 parts by mass based on 100 parts by mass of the addition reaction type liquid silicone resin.

The liquid epoxy resin means a liquid resin having a reactive epoxy group and having curability through a reaction with a curing agent of every kind. The liquid epoxy resin is not particularly limited, known raw materials can be broadly used, and any of experimental synthetic products and commercially available products may also be used. However, those having a long working life and having flexibility after curing are preferred. Examples of such a liquid epoxy resin include a rubber-modified epoxy resin and a long-chain epoxy resin.

Examples of the liquid epoxy resin having flexibility after curing as mentioned above include "EPICLON EXA-4816" and "EPICLON EXA-4850", all of which are available from DIC Corporation.

Although the curing agent of the liquid epoxy resin is not particularly limited, one which does not impair the flexibility of the cured product is preferred. As such a curing agent, known raw materials can be broadly used, and any of experimental synthetic products and commercially available products may also be used. Examples thereof include "LUCKAMIDE EA-330" and "LUCKAMIDE TD-984", all of which are available from DIC Corporation.

Although a blending amount of the curing agent is not particularly limited, it can be calculated on the basis of an epoxy equivalent of the liquid epoxy resin and an active hydrogen equivalent of the curing agent. Here, the epoxy equivalent means a numerical value expressing a molecular weight of the epoxy resin containing 1 equivalent of the epoxy group, and the active hydrogen equivalent means a numerical value expressing a molecular weight of the curing agent containing 1 equivalent of active hydrogen participating in the curing reaction. It is preferred to set the blending amount of the curing agent such that the amount of active hydrogen participating in the curing reaction is 0.8 to 1.2 equivalents to 1 equivalent of the epoxy group contained in the liquid epoxy resin. By allowing the blending amount of the curing agent to fall within the aforementioned range, a favorable cured product can be provided.

In particular, the liquid resin which is used for the backing material is preferably one having flexibility after curing. According to such a resin having flexibility, it can also be used upon being bent in conformity with the shape of the probe.

As the magnetic substance particle, it is not particularly limited so long as it is a magnetic substance particle capable of becoming a magnetized particle having a magnetic flux density of 1,000 to 15,000 gauss.

In this specification, the "magnetic substance particle" refers to a substance capable of becoming magnetized and refers to a substance which may become a magnetized particle after magnetization. For that reason, here, it should be construed that in the case of referring to the "magnetic substance particle", it means a particle not magnetized, namely a particle which does not become magnetic.

As such a magnetic substance particle, any of experimental synthetic products and commercially available products may be used. Examples thereof include a particle of iron, cobalt, nickel, or an alloy thereof, ferrite, or the like. These magnetic substance materials may be used alone or may be used in combination of two or more thereof.

Examples of the ferrite particle include Ni-Zn-based ferrite and Mn-Zn-based ferrite. Examples of the commercially available product of such a ferrite particle include "KNI-106", "KNI-106GMS, "KNI-106GS", and "LD-M", all of which are available from JFE Chemical Corporation.

A residual magnetic flux density of such a magnetic substance particle is preferably 1,000 to 15,000 gauss, more preferably 1,100 to 10,000 gauss, and still more preferably 1,200 to 5,000 gauss. By using the magnetic substance particle having the aforementioned residual magnetic flux density, in a step as mentioned later, when impressing a magnetic field on a molded body, the magnetic particle contained in the molded body can be changed to a magnetized particle having desired magnetic flux density.

The residual magnetic flux density of the magnetic particle is a residual magnetic flux density as a physical properties value. This value is adopted as a value of the residual magnetic flux density described in a product catalog of magnetic substance particle, and in the case where the value of residual magnetic flux density is not available from the foregoing catalog or the like, a value measured by a known method may also be adopted.

An average particle diameter of the magnetic substance particle is preferably 0.1 to 90 µm, and more preferably 0.8 to 90 µm. In the present invention, even when the filler particle is a small-diameter particle, high attenuation characteristics are obtained, and therefore, the scattering in the attenuation effect of the acoustic wave vibration between the elements can be reduced while maintaining the attenuation characteristics well. The average particle diameter means a value as measured by the method described in the section of Examples.

A density of the magnetic substance particle is preferably 3.0 to 9.0 g/cm³, and more preferably 5.0 to 9.0 g/cm³. Such a magnetic substance particle is able to efficiently attenuate the acoustic wave vibration as the high-density particle. The density of the magnetic substance particle refers to a true density (catalog value) inherent to a material, and in the case where the value of true density is not available from the product catalog of magnetic substance particle, or the like, a value measured by a known method may also be adopted.

Examples of the other component include a coloring agent, a platinum catalyst, a curing accelerator, a curing retarder, a solvent, a dispersant, an antistatic agent, an antioxidant, a flame retarder, and a thermal conductivity enhancer. As for all of the materials, known materials can be broadly used, and any of experimental synthetic products and commercially available products may also be used. In addition, these components may be used alone or may be used in combination of two or more thereof.

Although a blending amount of the coloring agent is not particularly limited, it is preferably 0.01 to 10 parts by mass, and more preferably 0.01 to 5 parts by mass based on 100 parts by mass of the liquid resin. In addition, though a blending amount of the curing accelerator is not particularly limited, it is preferably 0.1 to 20 parts by mass based on 100 parts by mass of the liquid resin.

Subsequently, the respective components thus prepared are mixed to prepare a resin composition. In the present invention, in particular, by mixing the aforementioned liquid resin and the magnetic substance particle, workability and moldability become favorable.

A mixing method is not particularly limited, and the mixing can be performed by a known method. Examples of such a mixing method include methods, such as kneading with a roll mill, a kneader, or the like, agitation with an impeller, and agitation with a planetary type agitation mixing machine. The resin composition may be subjected to a degassing treatment as mentioned later, as the need arises.

### (Step of Curing the Resin Composition)

The thus obtained resin composition is molded in a predetermined shape and cured.

A molding method is not particularly limited, and the molding can be performed by a known method. Examples thereof include a method in which the mixed resin composition is poured into a molding die, clamped, and then cured. In addition, a molding shape is not particularly limited, too, the resin composition may be formed into a desired shape according to a use mode or the like, and the cured product may be formed into a predetermined shape through post-processing (shape processing, for example, cutting, machining, and grinding).

A curing method is not particularly limited, and it varies with a material system. For example, it is preferred that the curing is performed under the following condition.

In the case of thermal curing, a treatment temperature is preferably 50 to 150°C, and more preferably 70 to 150°C. By allowing the treatment temperature to fall within the aforementioned range, not only the curing can be performed without taking time, but also dimensional accuracy is readily obtained.

A curing time is preferably 0.5 to 5.0 hours, and more preferably 0.5 to 3.0 hours. By allowing the curing time to fall within the aforementioned range, a backing material having a strength required in practical use can be provided.

Because the resin composition occasionally contains an air bubble in the production process, in the case where a molded article having less bubbles is desired, it is preferred to perform a degassing treatment. The degassing treatment can be performed by a known method, and examples thereof include vacuum degassing and agitation degassing.

### (Step of Impressing a Magnetic Field on the Cured Product)

A magnetic field is impressed on the thus obtained cured product of the resin composition. According to this, the magnetic substance particle dispersed in the cured product is magnetized to become a magnetized particle having a desired magnetic flux density. In the thus obtained cured product after magnetization (backing material), in view of the fact that the magnetized particles magnetically interact with each other, an excellent attenuation effect of acoustic wave vibration is exhibited.

In order to improve the dispersion of the magnetic substance particle, it is desired that before curing of the resin composition, the magnetic substance particle is existent in a state where it is not strongly magnetized. When the magnetic substance particle has been strongly magnetized in a state before curing, there is a concern that the dispersibility of particle as the filler particle is worsened such that the magnetic interaction largely works between the particles, thereby causing aggregation of particles in the resin composition, or the like.

A method of impressing a magnetic field in order to magnetize the magnetic substance particle is not particularly limited, and the magnetization can be performed by a known method. Examples thereof include a pulse system by a high-voltage capacitor; and a non-power supply magnetization method using a rare earth metal. In particular, it is preferred that such impression of a magnetic field is performed until thoroughly reaching a saturated magnetic flux density of the magnetic substance particle. The magnetic substance particle having been impressed with a magnetic field becomes a magnetized particle having a magnetic flux density substantially corresponding to the foregoing saturated magnetic flux density.

### (Other Step)

The aforementioned production method may include other step than the aforementioned steps, as the need arises. It is possible to conduct various treatments for improving chemical resistance, waterproofness, abrasion resistance, adhesiveness, and so on within a range where the attenuation effect of acoustic wave vibration is not influenced.

### [Acoustic Wave Probe]

The backing material of the present invention is suitably used as a structural member of an acoustic wave probe.

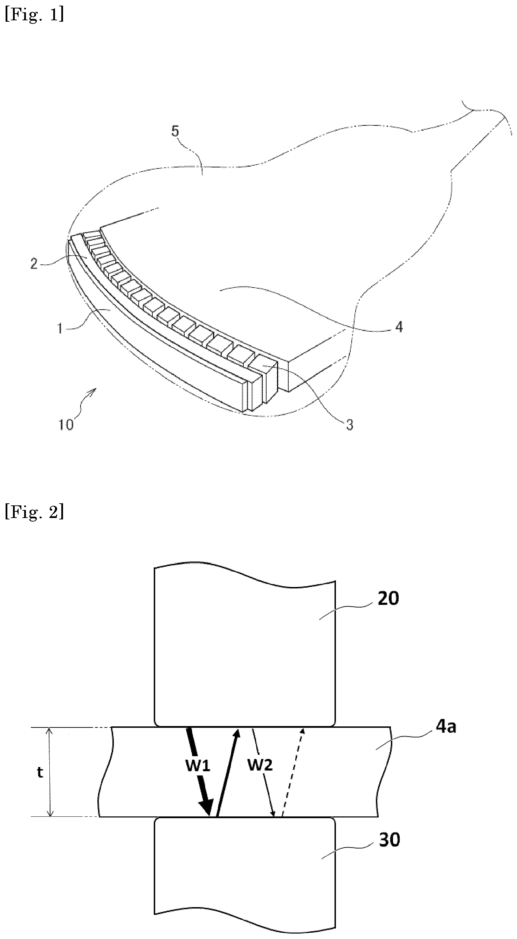

A representative structure of an acoustic wave probe is shown in Fig. 1 in terms of a diagrammatic perspective view (partial transparent view). An acoustic wave probe 10 shown in Fig. 1 is provided with an acoustic lens 1, an acoustic matching layer 2, a piezoelectric element (transducer) 3, and a backing 4 in this order from on the ultrasonic transmission/reception surface side (object side), and further provided with a casing 5 accommodating these elements.

In the acoustic probe 10 provided with the backing material 4 of the present invention, because an acoustic wave is efficiently absorbed in the interior of the backing material 4, by acoustically braking it to suppress an excessive vibration, a pulse interval of the ultrasonic waves can be shortened, and a distance resolution in an ultrasonic diagnostic image can be improved. Thus, it becomes possible to perform ultrasonic diagnosis by a shape image.

While the embodiments of the present invention have been described, it should be construed that the present invention is not limited to the aforementioned embodiments. The present invention includes all aspects included in the concept of the present invention and appended claims, and various modifications can be made within the scope of the present disclosure.

### Examples

The present invention is hereunder described in more detail by reference to Examples. However, it should be construed that the present invention is by no means limited to the following Examples.

With respect to the Examples and Comparative Examples as mentioned later, the respective evaluations were performed under the following conditions.

### [1] Average Particle Diameter

The average particle diameter of the magnetic substance particle was measured using a laser diffraction particle size distribution analyzer (a trade name: LA-500, available from Horiba, Ltd.).

Specifically, the magnetic substance particle was added in water having a surfactant added thereto and subjected to an ultrasonic treatment to thoroughly disperse the magnetic substance particle. Then, this slurry was used as a measurement sample and measured for particle size distribution by the aforementioned analyzer. In a cumulative particle size distribution of the obtained magnetic substance particle, a particle diameter (D50) of cumulative percentage 50% was defined as the average particle diameter.

### [2] Density

The density was calculated from a mass of the sample in air and water by collecting gas over water according to the following expression (1). $Density of sample = {W}_{a} / \left({W}_{a}-{W}_{1}\right) \times {ρ}_{1}$ (1)

In the expression (1), Wₐ is a mass of the sample in air; W₁ is a mass of the sample in water; and ρ₁ is a density of water at room temperature (20°C ± 5°C).

### [3] Attenuation Effect

The attenuation effect was evaluated by the following method. The evaluation method is hereunder explained by reference to a diagrammatic view of Fig. 2.

A backing material prepared in each of the Examples and Comparative Examples was designated as a measurement sample 4a; as shown in Fig. 2, a transmitting frequency of 10 MHz was made incident into the sample 4a by using a transmitting probe 20; the intensity of each of a first wave W1 and a second wave W2 as observed by a reception probe 30 on the opposite surface to the incident surface of an ultrasonic wave was determined; and an attenuation factor was calculated according to the following expression (2) $Attenuation factor = 20 log \left(I1/I2\right) / 2 t$ (2)

In the expression (2), I1 and I2 are intensities of the first wave W1 and the second wave W2, respectively as observed by the reception probe 30; and t is a thickness [mm] of the backing material.

For the transmitting probe 20 and the reception probe 30, a probe for transmitting frequency of 10 MHz (a trade name: V127-RM, available from Olympus Corporation) was used.

In the Examples, the sample having an attenuation factor of 6.0 or more was evaluated as "A"; the sample having an attenuation factor of less than 6.0 and 4.5 or more was evaluated as "B"; and the sample having an attenuation factor of less than 4.5 was evaluated as "C". It is meant that the material having a large attenuation factor of acoustic wave vibration can be suitably used as the backing material.

### [4] Scattering of Attenuation Effect

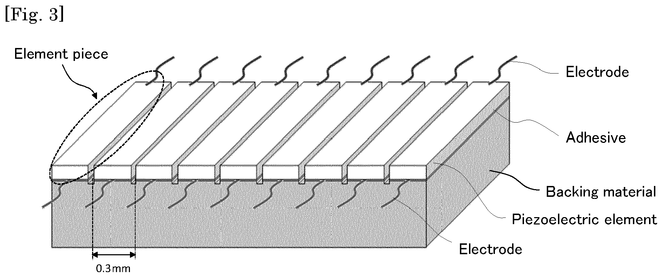

The scattering of the attenuation effect was evaluated by the following method. The evaluation method is hereunder explained by reference to a diagrammatic view of Fig. 3.

First of all, a piezoelectric element was laminated on a backing material prepared in each of the Examples and Comparative Examples via an adhesive, to obtain a laminate. Subsequently, as shown in Fig. 3, as for this laminate, the piezoelectric element was diced at a pitch of 0.3 mm until it reached the backing material, thereby cutting and dividing the piezoelectric element. Electrodes were attached to every cut and divided element, thereby preparing element pieces on the backing material.

Subsequently, 100 pieces arbitrarily selected among the aforementioned elements were each impressed with a predetermined voltage. At this time, an intensity of a main signal obtained from the element piece and an intensity of a signal of unnecessary vibration of the backing material were measured using an oscilloscope (Model No.: TBS1072B, available from Tektronix, Inc.), and a rate (%) of the signal intensity of the unnecessary vibration to the main signal intensity was calculated. From the thus determined rates (N = 100) of the 100 pieces, average value, maximum value, and minimum value thereof were determined.

In the Examples, the case where all of the maximum value and the minimum value of the aforementioned rates of the 100 pieces fall within the range of ±3% with respect to the average value was evaluated as "A"; the case where at least one of the maximum value and the minimum value of the aforementioned rates of the 100 piece was outside the range of ±3% with respect to the average value and inside the range of ±5% with respect to the average value was evaluated as "B"; and the case where at least one of the maximum value and the minimum value of the aforementioned rates of the 100 piece was outside the range of ±5% with respect to the average value was evaluated as "C".

The signal of the unnecessary vibration expresses excessive vibration which could not be completely suppressed by the backing material. For that reason, the scattering of the attenuation effect of the backing material can be confirmed by a difference in signal intensity of unnecessary vibration for every element piece.

### (Example 1)

An addition reaction type liquid silicone resin (a trade name: EG-3100, available from Dow Corning Toray Co., Ltd., viscosity: 0.4 Pa·s at room temperature (20°C ± 5°C), a curing agent (a trade name: RD-7, available from Dow Corning Toray Co., Ltd.), and a ferrite particle as a magnetic particle (a trade name: KNI-106, available from JFE Chemical Corporation; residual magnetic flux density (catalog value): 2,500 gauss, average particle diameter: 0.8 µm) were blended in predetermined proportions and subjected to a kneading treatment, to obtain a resin composition.

Here, in the aforementioned resin composition, the blending proportion of the curing agent was set to 1 part by mass based on 100 parts by mass of the addition reaction type liquid silicone resin, and the blending proportion of the ferrite particle was set to 567 parts by mass based on 100 parts by mass of the total amount of the addition reaction type liquid silicone resin and the curing agent.

The thus obtained resin composition was thermally cured at 120°C for 2 hours, to prepare a molded article of 20 mm × 80 mm and 2 mm in thickness. Thereafter, the obtained molded article was fixed within an air core inductor having an inside diameter of 50 mm, and a backing material magnetized at an impression voltage of 2,000 V by a capacitor type magnetizing power supply was prepared. Using this backing material, the aforementioned various evaluations were performed. It should be construed that the magnetic flux density and the average particle diameter of the magnetized particle contained in the backing material are corresponding to the residual magnetic flux density and the average particle diameter of the used magnetic particle. The results are shown in Table 1.

### (Example 2)

A liquid epoxy resin (a trade name: EPICLON EXA-4850, available from DIC Corporation, viscosity: 17.5 Pa·s at room temperature (20°C ± 5°C), epoxy equivalent: 440), a curing agent (a trade name: LUCKAMIDE EA-330, available from DIC Corporation, viscosity: 3.3 Pa·s at room temperature (20°C ± 5°C), active hydrogen equivalent: 95), and a ferrite particle as a magnetic particle (a trade name: KNI-106GSM (trademark), available from JFE Chemical Corporation; residual magnetic flux density (catalog value): 2,500 gauss, average particle diameter: 20 µm) were blended in predetermined proportions and subjected to a kneading treatment, to obtain a resin composition.

Here, in the aforementioned resin composition, as for the blending proportions of the liquid epoxy resin and the curing agent, the blending proportion of the curing agent was set to 18 parts by mass relative to 82 parts by mass of the liquid epoxy resin; and the blending proportion of the ferrite particle was set to 511 parts by mass based on 100 parts by mass of the total amount of the liquid epoxy resin and the curing agent.

In Example 2, a backing material was obtained by the same method as in Example 1, except that the resin composition was prepared in the manner as mentioned above.

### (Examples 3 and 4 and Comparative Examples 1 to 3)

In Examples 3 and 4 and Comparative Examples 1 to 3, backing materials were obtained by the same method as in Example 1, except that the following ferrite particles were used, respectively in place of the ferrite particle used in Example 1.
- Example 3: Ferrite particle (a trade name: KNI-106GS, available from JFE Chemical Corporation) having a residual magnetic flux density (catalog value) of 2,500 gauss and an average particle diameter of 90 µm
- Example 4: Ferrite particle (a trade name: LD-M, available from JFE Chemical Corporation) having a residual magnetic flux density (catalog value) of 1,300 gauss and an average particle diameter of 12 µm
- Comparative Example 1: Ferrite particle (a trade name: LD-MH, available from JFE Chemical Corporation) having a residual magnetic flux density (catalog value) of 760 gauss and an average particle diameter of 12 µm
- Comparative Example 2: Ferrite particle (a trade name: KNI-109, available from JFE Chemical Corporation) having a residual magnetic flux density (catalog value) of 800 gauss and an average particle diameter of 0.8 µm
- Comparative Example 3: Ferrite particle (a trade name: KNI-109GS, available from JFE Chemical Corporation) having a residual magnetic flux density (catalog value) of 800 gauss and an average particle diameter of 100 µm

### (Comparative Example 4)

In Comparative Example 4, a backing material was obtained by the same method as in Example 3, except that the magnetic field was not impressed on the molded article. That is, the backing material of Comparative Example 4 is the same as the molded article before magnetization as prepared in Example 3.

**Table 1**

| | Magnetized particle | | Density | Characteristics evaluation | |
|---|---|---|---|---|---|
| | Magnetic flux density | Average particle diameter | | Attenuation effect | Scattering of attenuation effect |
| | [G] | [µm] | [g/cm³] | | |
| Example 1 | 2,500 | 0.8 | 3.9 | A | A |
| Example 2 | 2,500 | 20 | 4.1 | A | A |
| Example 3 | 2,500 | 90 | 3.9 | A | B |
| Example 4 | 1300 | 12 | 3.9 | A | A |
| Comparative Example 1 | 760 | 12 | 3.9 | C | A |
| Comparative Example 2 | 800 | 0.8 | 3.9 | C | A |
| Comparative Example 3 | 800 | 100 | 3.9 | B | C |
| Comparative Example 4 | - | *90 | 3.9 | C | B |

| | | | | | |
|---|---|---|---|---|---|
| *: The value of the average particle diameter of Comparative Example 4 is a value of the non-magnetized magnetic substance particle. | | | | | |

As shown in Table 1, it was confirmed that the backing material containing the magnetized particle having a magnetic flux density falling within a range of 1,000 to 15,000 gauss is excellent in the attenuation effect of acoustic wave vibration (Examples 1 to 4).

In contrast, it was confirmed that as compared with the backing materials of Examples 1 to 4, in the case where the magnetic flux density of the magnetized particle contained in the backing material is less than 1,000 gauss, the attenuation effect of acoustic wave vibration is inferior (Comparative Examples 1 to 3).

In the non-magnetized magnetic substance particle, the magnetic interaction does not work a magnetic interaction between the particles, and thus, it was confirmed that as compared with the backing materials of Examples 1 to 4, the attenuation effect of acoustic wave vibration is inferior (Comparative Example 4).

In addition, according to the present invention, even in the case where the average particle diameter of the magnetized particle is 90 µm or less, a sufficient attenuation effect of acoustic wave vibration is obtained, and in particular, in the case where the average particle diameter of the magnetized particle is 20 µm or less, it was confirmed that the scattering with respect to the attenuation effect between the elements is less (Examples 1, 2, and 4).

### Reference Signs List

- 1:: Acoustic lens
- 2:: Acoustic matching layer
- 3:: Piezoelectric element
- 4:: Backing material
- 4a:: Measurement sample
- 5:: Casing
- 10:: Acoustic wave probe
- 20:: Transmitting probe
- 30:: Reception probe

## Claims

1. A backing material comprising a resin and a magnetized particle, wherein
the magnetized particle has a magnetic flux density of 1,000 to 15,000 gauss.

2. The backing material according to claim 1, wherein the magnetized particle has an average particle diameter of 0.1 to 90 pm.

3. The backing material according to claim 1 or 2, wherein the magnetized particle is ferrite.

4. An acoustic wave probe comprising the backing material according to any one of claims 1 to 3.

5. A method of producing a backing material, comprising
a step of obtaining a resin composition containing a liquid resin and a magnetic substance particle,
a step of curing the resin composition to obtain a cured product, and
a step of impressing a magnetic field on the cured product, to convert the magnetic substance particle into a magnetized particle, wherein
the magnetized particle has a magnetic flux density of 1,000 to 15,000 gauss.

6. The method of producing a backing material according to claim 5, wherein the magnetic substance particle has a residual magnetic flux density of 1,000 to 15,000 gauss.
